# DEMANDE DE BREVET EUROPEEN

(11) **EP 3 922 237 A1**
(43) Date de publication de la demande: **15.12.2021**
(21) Numéro de dépôt: 21178966.4
(22) Date de dépôt: 11.06.2021
(51) Int. Cl.: A61K 9/00, A61K 47/38

(54) **COMPOSITION ORODISPERSIBLE SOUS FORME D'UN COMPRIMÉ FONDANT / SOUS FORME D'UN COMPRIMÉ ORODISPERSIBLE COMPRENANT AU MOINS UNE VITAMINE COMME SUBSTANCE ACTIVE**

(30) Priorité: 11.06.2020 BE 202005422
(71) Demandeur: Cole Pharmaceuticals, 1390 Grez-Doiceau (BE)
(72) Inventeur: VANDEPLAS, Jean-Marie, 1390 Grez-Doiceau (BE); LUBICKI, Claude, 1180 Bruxelles (BE)
(74) Mandataire: ABYOO

(57) **Abrégé**

La présente invention porte sur une composition sous forme d'un comprimé fondant / sous forme d'un comprimé orodispersible comprenant au moins une vitamine comme substance active, et de la cellulose microcristalline comme agent épaississant pharmaceutiquement acceptable, ladite au moins une vitamine comme substance active étant présente à raison de 1% à 10% en poids par rapport au poids total de la composition, de préférence présente à raison de 2% à 5% en poids par rapport au poids total de la composition.

## Description

La présente invention porte sur une composition orodispersible sous forme d'un comprimé fondant / sous forme d'un comprimé orodispersible comprenant au moins une vitamine comme substance active.

Préférenbtiellement, la présente invention porte sur une composition orodispersible sous forme d'un comprimé fondant / sous forme d'un comprimé orodispersible comprenant au moins une vitamine D comme substance active, préférentiellement de la vitamine D3 (cholécalciférol) comme substance active.

Un comprimé fondant / comprimé orodispersible est une forme orodispersible présentant les principaux intérêts suivants : pas de risque d'étouffement (fausse route) pour les personnes âgées, les enfants, les patients atteints de maladie(s) mentale(s) ou les personnes alitées ; plus de confort au patient (pas de déglutition forcée) ; aucun apport de liquide n'est nécessaire pour être assimilé par l'individu ; une absorption buccale / sublinguale favorise une absorption plus rapide ; le dosage prescrit par le médecin est parfaitement respecté (ce n'est pas le cas de préparations huileuses actuellement vendue en flapule plastique avec un problème physique de viscosité) ; bon goût (masquage par des arômes fruités) par rapport aux formulations huileuses ; pas d'absorption inefficace par les intestins propre aux formes orales actuellement disponibles (comprimés à avaler) ; transport et stockage aisés.

Les comprimés orodispersibles ou comprimés fondants sont des formes de dosage solides contenant des substances actives (par exemple des médicaments) qui se désintègrent dans la cavité buccale en moins de 3 minutes, de préférence en moins d'une minute, laissant un résidu facile à avaler. En ce sens, la Pharmacopée européenne a adopté le terme de « comprimé orodispersible » pour désigner un comprimé qui se disperse ou se désintègre dans un délai inférieur à 3 minutes dans la bouche avant d'être avalé (orodispersibilité inférieure à 3 minutes).

De façon générale, il est reconnu qu'un comprimé orodispersible est choix opportun pour l'administration de substances actives (par exemple de médicaments) aux patients pédiatriques et gériatriques, car il permet de résoudre le problème de la dysphagie. En particulier, un comprimé fondant / comprimé orodispersible répond aux besoins de certains groupes de patients qui ont de graves problèmes de dysphagies comme par exemple les personnes âgées, les personnes alitées ou encore les personnes souffrant des maladies neuro-dégénératives. Les personnes âgées sont les utilisateurs de médicaments qui courent le plus grand risque de rencontrer des problèmes pratiques d'utilisation, ce qui peut augmenter le risque de mauvaise observance. L'importance d'une galénique parfaitement adaptée aux personnes âgées mais également aux enfants est cruciale, ce qui est possible avec des comprimés fondants / comprimés orodispersibles : flexibilité du dosage, facilité d'ingestion, risque d'étouffement réduit ou nul, portabilité adéquate, alternative à la formulation liquide orale, à des capsules dures ou à des comprimés classiques.

Dans l'état de la technique, le document EP0922464 divulgue un comprimé orodispersible pouvant comprendre une vitamine comme substance active. Il existe également le produit Vista-D3^{®} qui se présente sous la forme d'un comprimé fondant orodispersible dont les constituants sont les suivants : mannitol et cellulose comme diluants ; polyvinylpolypyrrolidone comme agent épaississant ; vitamine D3 (cholécalciferol) comme substance active ; stéarate de magnésium végétal et dioxyde de silicium colloïdal comme agents d'enrobage ; glycosides de stéviol comme édulcorant ; menthe comme arôme.

Dès lors que du polyvinylpolypyrrolidone est utilisé en tant qu'agent épaississant, le procédé de fabrication à mettre en œuvre pour obtenir de tels comprimés fondants / comprimés orodispersibles est la granulation par voie humide (le polyvinylpyrrolidone est utilisé pour une granulation humide puisqu'il n'est pas efficace pour une fixation à sec). La granulation par voie humide consiste à ajouter une solution liquide à des poudres, afin de former une masse à partir du mélange de particules de poudre séchée primaire à l'aide de liquide de granulation. En général, les liquides ajoutés sont de l'eau, de l'éthanol ou de l'isopropanol, seuls ou associés les uns aux autres. La solution liquide peut être à base aqueuse ou à base de solvant. L'eau mélangée aux poudres forme des liens entre les particules de poudre, ces liens devant être suffisamment forts pour assurer que les particules restent solidaires.

Malheureusement, le procédé de fabrication par granulation par voie humide présente certaines contraintes non négligeables :
- il s'agit d'un procédé reposant sur plusieurs étapes successives, ce qui augmente la complexité de la validation et du contrôle ;
- il s'agit d'un procédé coûteux en temps ;
- il s'agit d'un procédé pouvant donner lieu à des problèmes de stabilité, notamment pour des substances actives thermolabiles et/ou sensibles à l'humidité ;
- il s'agit d'un procédé où s'observent des pertes de matière au cours des différentes étapes du process.

Aussi, il est reconnu que le polyvinylpolypyrrolidone, un polymère organique de synthèse, contient des traces monomériques vraisemblablement cancérigènes, ce qui est particulièrement préoccupant pour la formulation de médicaments ou de compléments alimentaires.

A ce jour, il existe donc un réel besoin de procurer une composition orodispersible alternative sous forme d'un comprimé fondant / sous forme d'un comprimé orodispersible répondant aux critères de la Pharmacopée européenne en termes d'orodispersibilité (dispersion / désintégration / fonte dans un délai inférieur à 3 minutes) et comprenant au moins une vitamine comme substance active, cette composition orodispersible alternative étant sans danger pour la santé, pouvant être obtenue plus aisément, c'est-à-dire pouvant être obtenue sans avoir à recourir au procédé de granulation par voie humide, ceci tout en garantissant l'obtention de comprimés fondants / comprimés orodispersibles au moins équivalents à ceux obtenus par granulation par voie humide, notamment en termes de temps requis pour observer une fonte / dispersion / désagrégation / désintégration totale du comprimé.

Pour adresser au moins en partie ces problèmes, il est prévu, suivant l'invention, une composition orodispersible sous forme d'un comprimé fondant / sous forme d'un comprimé orodispersible comprenant au moins une vitamine comme substance active et de la cellulose microcristalline comme agent épaississant, ladite composition étant caractérisée en ce que ladite au moins une vitamine comme substance active est présente à raison de 1% à 10% en poids par rapport au poids total de la composition, de préférence présente à raison de 2% à 5% en poids par rapport au poids total de la composition.

La cellulose microcristalline (MCC) est un additif naturel de la famille de la cellulose, référencée E460. Il s'agit d'un excipient naturel extrait de fibre végétale, très utilisé dans les industries agro-alimentaire et pharmaceutique, notamment comme liant / diluant / épaississant dans des formulations de comprimés oraux et de capsules. La cellulose microcristalline a une densité gonflante minimale, augmentant les caractéristiques de fluidité et assurant que chaque ingrédient est uniformément réparti au sein d'une mixture. Si le mélange est consistant, chaque comprimé aura la même quantité de chaque ingrédient au terme de la production, créant un produit de qualité. En outre, la cellulose microcristalline est directement compressible et par conséquent peut être compressée dans un comprimé sans avoir à broyer ou à traiter un mélange. De plus, la cellulose microcristalline est sans danger pour une consommation en quantités normales, et est d'ailleurs largement utilisée comme excipient pour la fabrication de comprimés. Les tests pratiqués *in vitro* chez des souris n'ont indiqué aucun effet génotoxique. La cellulose microcristalline ne provoque aucune réaction métabolique, d'où l'absence d'effets néfastes pour l'organisme. Enfin, la cellulose microcristalline est une source naturelle de fibre alimentaire sans calories et est inerte, c'est-à-dire qu'elle ne réagira pas ou n'interférera pas avec d'autres substances.

Par les termes « substance active », il est entendu, au sens de la présente invention, un principe actif ou un ingrédient actif entrant dans la composition d'un médicament ou d'un complément alimentaire et qui possède un effet thérapeutique ou préventif.

Par les termes « comprimé fondant » / « comprimé orodispersible », il est entendu, au sens de la présente invention, une forme pharmaceutique (par exemple un médicament) ou une forme de complément alimentaire solide orodispersible destinée à la voie orale et se désagrégeant / fondant / se dispersant dans la bouche (dans la cavité buccale) au contact de la salive pour y libérer la substance active, cette forme pharmaceutique (par exemple un médicament) ou cette forme de complément alimentaire solide pouvant contenir une ou plusieurs substances actives. Au sens de la présente invention, un comprimé fondant / comprimé orodispersible se désintègre ou se disperse dans la cavité buccale dans un délai inférieur à 3 minutes avant d'être avalé, de préférence dans un délai inférieur à une minute environ avant d'être avalé.

En particulier, selon l'invention, les termes « comprimé fondant » / « comprimé orodispersible » désignent une telle forme pharmaceutique (par exemple un médicament) ou une telle forme de complément alimentaire solide équivalant à une dose (unité de prise) d'une substance active donnée.

Par les termes « composition orodispersible », il est entendu, au sens de la présente invention, une composition qui se disperse / fond / se désagrège dans la bouche, par exemple qui se dissout dans la bouche par simple contact avec la salive.

Dans le cadre de la présente invention, il a été mis en évidence que, lorsque de la cellulose microcristalline est mise en œuvre en tant qu'agent épaississant, des comprimés fondants / comprimés orodispersibles sans risque pour la santé des consommateurs peuvent être obtenus au travers d'un procédé de granulation par voie sèche (agglomération par compression d'un volume prédéterminé de particules sous forme de poudre ou des granule), les comprimés fondants / comprimés orodispersibles obtenus par voie sèche présentant des propriétés au moins équivalentes à celles observées avec des comprimés fondants / comprimés orodispersibles obtenus par voie humide. Les comprimés fondants / comprimés orodispersibles suivant la présente invention permettent donc de s'affranchir non seulement des contraintes rencontrées avec un procédé de fabrication par granulation par voie humide mais aussi de tout risque pour la santé des consommateurs.

Par ailleurs, il a été déterminé qu'une composition selon l'invention sous forme d'un comprimé fondant / comprimé orodispersible comprenant au moins une vitamine comme substance active présente à raison de 1% à 10% en poids par rapport au poids total de la composition, de préférence présente à raison de 2% à 5% en poids par rapport au poids total de la composition, et de la cellulose microcristalline comme agent épaississant se désagrège / fond / se disperse dans un délai inférieur à 3 minutes, voire dans un délai de l'ordre de la minute, voire dans un délai inférieur à la minute, voire même dans un délai de quelques secondes, dans la bouche sans ajout de liquide autre que la salive, cette désintégration rapide assurant un certain confort pour le patient et permettant de répondre aux critères de la Pharmacopée européenne en termes d'orodispersibilité (dispersion / désintégration / fonte dans un délai inférieur à 3 minutes).

Un comprimé fondant / comprimé orodispersible selon l'invention permet également de prendre le comprimé sans risque de fausse route ni d'étouffement contrairement à certaines formes orodispersibles où ces problématiques sont régulièrement rencontrées.

De préférence, selon l'invention, ledit comprimé fondant / comprimé orodispersible se présente la forme d'un cylindre droit dont les faces inférieurs et supérieures sont convexes et les bords biseautés.

Selon un mode de réalisation préféré suivant l'invention, ledit comprimé fondant / comprimé orodispersible n'est pas sécable.

Avantageusement, selon l'invention, ladite au moins une vitamine est choisie dans le groupe constitué des vitamines D, des vitamines B, de la vitamine A, de la vitamine C, de la vitamine E, de la vitamine K1, de la vitamine K2 et leurs mélanges.

De préférence, selon l'invention, ladite au moins une vitamine est une vitamine D.

De préférence, selon l'invention, ladite au moins une vitamine est une vitamine B.

De préférence, selon l'invention, ladite au moins une vitamine est une vitamine A.

De préférence, selon l'invention, ladite au moins une vitamine est une vitamine C.

De préférence, selon l'invention, ladite au moins une vitamine est une vitamine E.

De préférence, selon l'invention, ladite au moins une vitamine est une vitamine K.

Parmi les vitamines B, citons par exemple les vitamines : B2 (rivoflvine), B3 (nacine), B5 acide pantothénique), B6 (pyroxidine), B8 (biotine), B9 (acide folique) et B12 (metylcolabamine).

Eventuellement, la composition selon l'invention, comprend un flavonoïde, par exemple la quercétine.

De préférence, selon l'invention, ladite au moins une vitamine est la vitamine D3. La vitamine D3 (cholécalciférol) est la forme naturelle de la vitamine D. Elle est essentiellement synthétisée par la peau (couches superficielles de l'épiderme) grâce au soleil (exposition aux rayons ultraviolets B), et est présente dans certains aliments. La vitamine D3 intervient dans la calcification en permettant notamment la fixation du calcium particulièrement nécessaire dès les premiers mois de la vie : elle est donc essentielle au développement normal du squelette. Cette vitamine participe au maintien des niveaux sanguins normaux de calcium et de phosphore, absorbés par l'intestin. Elle joue un rôle dans le maintien des muscles squelettiques et favorise la solidité des os. Son absence provoque des troubles du métabolisme du calcium et du phosphore. Un déficit en vitamine D3 est à l'origine de différentes pathologies osseuses, comme par exemple le rachitisme chez l'enfant, l'ostéomalacie chez l'adulte jusqu'à l'ostéoporose chez les adultes plus âgés. La vitamine D a aussi des effets non classiques ou extra squelettiques.

Il a été observé des associations entre l'apport de vitamine D et la réduction de la mortalité, de certains cancers, la réduction des risques d'infections incluant les infections virales comme le COVID-19, de maladies inflammatoires (diabète, sclérose en plaques) ou encore de maladies cardiovasculaires.

Notons que de faibles taux sériques de 25-hydroxy vitamine D [25(OH)D] sont des marqueurs de carence en vitamine D.

Selon l'invention, ladite au moins une vitamine comme substance active est présente à raison de 1% à 10% en poids par rapport au poids total de la composition, de préférence à raison de 2% à 5% en poids par rapport au poids total de la composition.

Avantageusement, selon l'invention, ladite au moins une vitamine comme substance active est présente en une quantité en poids inférieur à 10% par rapport au poids total de la composition.

Avantageusement, selon l'invention, ladite au moins une vitamine comme substance active est présente en une quantité en poids inférieur à 9% par rapport au poids total de la composition, de préférence en une quantité en poids inférieur à 8% par rapport au poids total de la composition, de préférence en une quantité en poids inférieur à 7% par rapport au poids total de la composition, de préférence en une quantité en poids inférieur à 6% par rapport au poids total de la composition, de préférence en une quantité en poids inférieur à 5% par rapport au poids total de la composition, de préférence en une quantité en poids inférieur à 4% par rapport au poids total de la composition, de préférence en une quantité en poids inférieur à 3% par rapport au poids total de la composition, de préférence en une quantité en poids inférieur à 2% par rapport au poids total de la composition, de préférence en une quantité en poids inférieur à 1% par rapport au poids total de la composition.

Avantageusement, selon l'invention, ladite cellulose microcristalline comme agent épaississant pharmaceutiquement acceptable est présente à raison de 5% à 25% en poids par rapport au poids total de la composition, de préférence à raison de 10% à 20% en poids par rapport au poids total de la composition.

Préférentiellement, la composition selon l'invention comprend en outre au moins un agent émulsifiant pharmaceutiquement acceptable, par exemple le crospovidone, les lécithines, les monoglycérides et les diglycérides d'acides gras et leurs mélanges. Les émulsifiants sont des molécules qui possèdent une extrémité ayant une affinité pour l'eau (hydrophile) et une extrémité ayant une affinité pour l'huile (hydrophobe). Grâce à eux, l'eau et l'huile peuvent être finement dispersées l'une dans l'autre pour créer une émulsion stable, homogène et fluide. Ainsi, un émulsifiant est un additif qui permet de créer une émulsion uniforme. Les émulsifiants sont utiles pour mixer et stabiliser des substances, notamment, les mélanges de matières grasses et d'eau en vue de les rendre miscibles entre elles. Les agents émulsifiants les plus utilisés sont les lécithines et les monoglycérides et diglycérides d'acides gras alimentaires.

De préférence, la composition selon l'invention comprend en outre au moins un agent édulcorant pharmaceutiquement acceptable, par exemple le mannitol, le xylitol, l'aspartame, la saccharine, l'acésulfame K, le sucralose, les extraits de stévia (glycosides de stéviol), le sucralose et leurs mélanges. Les édulcorants sont des produits ou des substances ayant un goût sucré. Le plus souvent, le terme « édulcorant » fait référence à des ingrédients destinés à changer le goût d'un aliment, d'un médicament ou d'un complément alimentaire en lui conférant une saveur sucrée. Certains édulcorants n'apportent pas de calories, d'autres moins que le sucre de table, d'autres ont l'avantage de ne pas être cariogènes et certains sont plus sucrés que le sucre.

Préférentiellement, la composition selon l'invention comprend en outre au moins un agent correcteur d'acidité pharmaceutiquement acceptable, par exemple l'acide citrique.

Avantageusement, la composition selon l'invention comprend en outre au moins un agent colorant pharmaceutiquement acceptable, par exemple un concentré de safran, un concentré de pomme, un concentré de cassis, un concentré de radis et leurs mélanges.

De préférence, la composition selon l'invention comprend en outre au moins un agent anti-agglomérant ou lubrifiant pharmaceutiquement acceptable, par exemple du stéarate de magnésium, de l'acide stéarique, du stéarate de calcium, de l'huile de colza hydrogénée, de la lécithine, du dibéhénate de glycéryle et leurs mélanges. Un anti-agglomérant ou lubrifiant est une substance qui limite l'agglutination des particules dans un produit en poudre et ainsi assure sa fluidité. Ces additifs limitent l'agglutination des particules ou la formation des agrégats par suite d'une exposition à l'humidité de l'air. Certains sont des produits naturels tels que le talc et la bentonite, d'autres sont généralement constitués d'amidon, de carbonate et de silice. Le carbonate de sodium (E 500), le ferrocyanure de potassium (E 536), le dioxyde de silicium (E 551), le silicate de calcium (E 551), la bentonite (E 558) et le talc (E 553-ii-) sont des agents antiagglomérants. Les agents antiagglomérants sont notamment incorporés dans les compléments alimentaires dans le but d'éviter la formation des grumeaux. Ils fonctionnent en absorbant l'eau ou l'excès d'humidité. Les agents antiagglomérants facilitent l'écoulement libre des préparations en poudre et limitent l'agglutination des particules.

Préférentiellement, la composition selon l'invention comprend en outre au moins un arôme de fruits, par exemple un arôme de fraise, d'orange, menthe ou encore de citron.

Avantageusement, la composition selon l'invention se présente sous forme d'un complément alimentaire ou d'un médicament.

La présente invention porte également sur une utilisation d'une composition suivant l'invention pour utilisation dans le traitement préventif et/ou curatif de maladies et/ou de pathologies en lien avec le système immunitaire, en lien avec le système osseux et en lien avec le système musculaire.

La présente invention porte également sur une utilisation d'une composition suivant l'invention pour pallier une carence en vitamine, en particulier pour pallier une carence en vitamine D, plus particulièrement pour pallier une carence en vitamine D3. En d'autres termes, la présente invention porte également sur une utilisation d'une composition suivant l'invention pour utilisation dans un traitement palliant une carence en vitamine, en particulier pour pallier une carence en vitamine D, plus particulièrement pour pallier une carence en vitamine D3.

En particulier, une composition selon l'invention contribue au fonctionnement normal du système immunitaire, au maintien d'une ossature normale, au maintien d'une dentition normale, à une croissance et un développement osseux normaux des enfants, au maintien d'une fonction musculaire normale et au maintien d'une calcémie normale.

Une composition selon l'invention est particulièrement et préférentiellement indiquée en cas de besoins accrus en vitamine D3 ou lorsque la synthèse de cette vitamine par l'organisme est réduite, notamment pendant les périodes peu ensoleillées (automne et hiver), chez les personnes âgées, hommes et femmes, surtout lorsqu'elles sortent peu, chez les sujets plus âgés, hommes et femmes, en raison d'une production réduite et donc de plus faibles réserves en vitamine D3 afin de maintenir une ossature solide et en périodes de forte croissance, pendant la grossesse et l'allaitement.

### Exemples

### Exemple 1 : composition selon l'invention se présentant sous forme d'un comprimé fondant / comprimé orodispersible

Le Tableau 1 ci-dessous est un exemple d'une composition selon l'invention se présentant sous forme d'un comprimé fondant / comprimé orodispersible. Dans cette composition selon l'invention, une vitamine, en l'occurrence de la vitamine D3, est présente à raison de 3,38% en poids par rapport au poids total de la composition.

**Tableau 1**

| Composé | Par comprimé fondant / comprimé orodispersible | Actifs pour 100g |
|---|---|---|
| Vitamine D3 | 8 mg | 3,72 g |
| Arôme (orange) | 20 mg | 8,45 g |
| Colorant orange | 10 mg | 4,22 g |
| Mannitol | 100 mg | 42,23 g |
| Xylitol | 40 mg | 16,89 g |
| Cellulose microcristalline | 30 mg | 12,67 g |
| Crospovidone | 20 mg | 8,45 g |
| Acide citrique | 5 mg | 2,11 g |
| Stéarate de magnésium | 3 mg | 1,27 g |

### Exemple 2 : essais comparatifs en termes d'orodispersibilité

Les compositions sous forme de comprimés reprises au Tableau 2 ci-dessous ont été testées en termes d'orodispersibilité (propriété orodispersible des compositions sous forme de comprimés). Les compositions 1 à 3 (Compo 1, Compo 2 et Compo 3) correspondent à des compositions selon la présente invention puisque la quantité en poids de vitamine (en l'occurrence de vitamine D3) comme substance active dans chacune de ces compositions est comprise entre 1% à 10% en poids par rapport au poids total de la composition. Par contre, la composition 4 (Compo 4) comprenant plus de 10% en poids de vitamine (en l'occurrence de vitamine D3) comme substance active par rapport au poids total de la composition ne correspond pas à une composition selon la présente invention.

**Tableau 2**

| | Quantité en poids par comprimé | | | |
|---|---|---|---|---|
| | Compo 1 | Compo 2 | Compo 3 | Compo 4 |
| Vitamine D3 | 4,4 mg | 8,8 mg | 22 mg | 30 mg |
| Arôme | 20 mg | 10 mg | 20 mg | 15 mg |
| Colorant | 10 mg | 10 mg | 10 mg | 10 mg |
| Mannitol | 100 mg | 100 mg | 100 mg | 98 mg |
| Xylitol | 40 mg | 40 mg | 40 mg | 40 mg |
| Cellulose microcristalline | 40 mg | 30 mg | 40 mg | 50 mg |
| Crospovidone | 20 mg | 20 mg | 20 mg | 20 mg |
| Acide citrique | 5 mg | 5 mg | 2 mg | 2 mg |
| Stéarate de magnésium | 3 mg | 3 mg | 3 mg | 3 mg |
| Poids total | 237,4 mg | 236,8 mg | 257 mg | 258 mg |
| % en poids de vitamine D3 | 1,85 | 3,72 | 8,56 | 11,63 |
| Orodispersibilité* | < 3 min | < 3 min | < 3 min | > 3 min |

| | | | | |
|---|---|---|---|---|
| temps (durée) en minutes (min) nécessaire pour assurer la désintègration / la dispersion / la fonte totale du comprimé fondant / comprimé orodispersible dans la cavité buccale | | | | |

Comme on peut le constater, les compositions selon l'invention (Compo 1, Compo 2 et Compo 3) sous forme de comprimés comprenant entre 1% et 10% en poids de vitamine comme substance active par rapport au poids total de la composition sont bien orodispersibles puisqu'une orodispersibilité (désintégration / fonte / dispersion dans la cavité buccale) totale des comprimés est observée dans un délai inférieur à 3 minutes et même dans un délai de l'ordre de la minute, ce qui répond bien aux critères de la Pharmacopée européenne quant à la notion de comprimé orodispersible / comprimé fondant.

Par contre, la composition Compo 4 sous forme d'un comprimé comprenant plus de 10% en poids de vitamine comme substance active par rapport au poids total de la composition n'est pas orodispersible puisqu'une orodispersibilité (désintégration / fonte / dispersion dans la cavité buccale) totale des comprimés n'est pas observée dans un délai inférieur à 3 minutes. Pour la Compo 4, le comprimé est qualifié de comprimé à croquer et non pas de comprimé fondant / comprimé orodispersible puisqu'il ne se dissout pas / ne fond pas / ne se disperse pas dans la cavité buccale dans un délai inférieur à 3 minutes.

De façon surprenante et inattendue, il a donc été mis en évidence, dans le cadre de la présente invention, qu'une composition selon l'invention sous forme d'un comprimé comprenant au moins une vitamine comme substance active présente à raison de 1% à 10% en poids par rapport au poids total de la composition, de préférence présente à raison de 2% à 5% en poids par rapport au poids total de la composition, et de la cellulose microcristalline comme agent épaississant est bien orodispersible puisque les comprimés se désagrègent / fondent / se dispersent dans un délai inférieur à 3 minutes dans la bouche sans ajout de liquide autre que la salive, ce qui n'est pas le cas pour des compositions comprenant plus de 10% en poids de vitamine comme substance active par rapport au poids total de la composition.

La présente invention a été décrite en relation avec des modes de réalisations spécifiques, qui ont une valeur purement illustrative et ne doivent pas être considérés comme limitatifs. D'une manière générale, il apparaîtra évident pour l'homme du métier que la présente invention n'est pas limitée aux exemples illustrés et/ou décrits ci-dessus. L'usage des verbes « comprendre », « inclure », « comporter », ou toute autre variante, ainsi que leurs conjugaisons, ne peut en aucune façon exclure la présence d'éléments autres que ceux mentionnés.

L'usage de l'article indéfini « un », « une », ou de l'article défini « le », « la » ou « l' », pour introduire un élément n'exclut pas la présence d'une pluralité de ces éléments.

## Revendications

1. Composition orodispersible sous forme d'un comprimé fondant / sous forme d'un comprimé orodispersible comprenant au moins une vitamine comme substance active, et de la cellulose microcristalline comme agent épaississant pharmaceutiquement acceptable, ladite composition étant **caractérisée en ce que** ladite au moins une vitamine comme substance active est présente à raison de 1% à 10% en poids par rapport au poids total de la composition, de préférence présente à raison de 2% à 5% en poids par rapport au poids total de la composition.

2. Composition selon la revendication 1, **caractérisée en ce que** ladite au moins une vitamine est choisie dans le groupe constitué des vitamines D, des vitamines B, de la vitamine A, de la vitamine C, de la vitamine E, de la vitamine K1, de la vitamine K2 et leurs mélanges.

3. Composition selon la revendication 1, **caractérisée en ce que** ladite au moins une vitamine est la vitamine D3.

4. Composition selon l'une quelconque des revendications précédentes, **caractérisée en ce que** ladite cellulose microcristalline comme agent épaississant pharmaceutiquement acceptable est présente à raison de 5% à 25% en poids par rapport au poids total de la composition, de préférence à raison de 10% à 20% en poids par rapport au poids total de la composition.

5. Composition selon l'une quelconque des revendications précédentes, **caractérisée en ce qu'**elle comprend en outre au moins un agent émulsifiant pharmaceutiquement acceptable, par exemple le crospovidone, les lécithines, les monoglycérides et les diglycérides d'acides gras et leurs mélanges.

6. Composition selon l'une quelconque des revendications précédentes, **caractérisée en ce qu'**elle comprend en outre au moins un agent édulcorant pharmaceutiquement acceptable, par exemple le mannitol, le xylitol, l'aspartame, la saccharine, l'acésulfame K, le sucralose, les extraits de stévia (glycosides de stéviol), le sucralose et leurs mélanges.

7. Composition selon l'une quelconque des revendications précédentes, **caractérisée en ce qu'**elle comprend en outre au moins un agent colorant pharmaceutiquement acceptable, par exemple un concentré de safran, un concentré de pomme, un concentré de cassis, un concentré de radis et leurs mélanges.

8. Composition selon l'une quelconque des revendications précédentes, **caractérisée en ce qu'**elle comprend en outre au moins un agent correcteur d'acidité pharmaceutiquement acceptable, par exemple l'acide citrique.

9. Composition selon l'une quelconque des revendications précédentes, **caractérisée en ce qu'**elle comprend en outre au moins un agent anti-agglomérant ou lubrifiant pharmaceutiquement acceptable, par exemple du stéarate de magnésium, de l'acide stéarique, du stéarate de calcium, de l'huile de colza hydrogénée, de la lécithine, du dibéhénate de glycéryle et leurs mélanges.

10. Composition selon l'une quelconque des revendications précédentes, **caractérisée en ce qu'**elle comprend en outre au moins un arôme de fruits, par exemple un arôme de fraise, d'orange, menthe ou encore de citron.

11. Composition selon l'une quelconque des revendications précédentes, ladite composition se présentant sous forme d'un complément alimentaire ou d'un médicament.

12. Composition selon l'une quelconque des revendications précédentes pour utilisation dans le traitement préventif et/ou curatif de maladies et/ou de pathologies en lien avec le système immunitaire, en lien avec le système osseux et en lien avec le système musculaire.

13. Composition selon l'une quelconque des revendications 1 à 11 pour utilisation dans un traitement palliant une carence en vitamine, en particulier pour pallier une carence en vitamine D, plus particulièrement pour pallier une carence en vitamine D3.
